# EUROPEAN PATENT APPLICATION

(11) **EP 3 779 425 A1**
(43) Date of publication of application: **17.02.2021**
(21) Application number: 19775781.8
(22) Date of filing: 25.01.2019
(51) Int. Cl.: G01N 27/327, G01N 27/416

(54) **BIOLOGICAL SUBSTANCE DETECTION SENSOR**

(30) Priority: 26.03.2018 JP 2018058552
(71) Applicant: PHC Holdings Corporation, Tokyo 105-8433 (JP)
(72) Inventor: HANEDA, Keigo, Ehime, 791-0395 (JP); HAYASHINO, Nao, Ehime, 791-0395 (JP)
(74) Representative: Eisenführ Speiser
(86) International application number: PCT/JP2019/002476
(87) International publication number: WO 2019/187575

(57) **Abstract**

Provided is a biological substance detection sensor used in an electrochemical measurement method and allowing high-precision measurement by reducing a blank current. A biological substance detection sensor of the present invention for analyzing a component in a testing solution using a mediator includes at least: an insulating substrate; a conductive part formed on a surface of the insulating substrate and including at least one pair of a working electrode and a counter electrode; and a reagent part disposed in contact with or in a vicinity of the conductive part and including at least one of protein and the mediator. The reagent part further includes at least one additive selected from the group consisting of a halide and/or a pseudo halide.

## Description

### TECHNICAL FIELD

The present invention relates to a biological substance detection sensor, and more particularly to a biological substance detection sensor, a biological substance detection measurement kit, and a method for measuring component which use an electrochemical measuring method.

### BACKGROUND ART

In various fields such as a medical field and a clinical inspection field, a biological substance detection sensor is used to measure a substance to be detected in a biological sample. As an example of the biological substance detection sensor, a biological substance detection sensor using an electrochemical measurement method has been known (for example, Patent Document 1). In the biological substance detection sensor, a working electrode, a counter electrode, and a reference electrode are formed on an insulating substrate, and an enzyme reaction layer (also referred to as a reagent part) including an enzyme and an electron acceptor (hereinafter referred to as a mediator) is formed in contact with these electrodes. According to such a biological substance detection sensor, various substances can be principally measured by selecting an enzyme including a substance to be measured as a substrate. For example, a glucose sensor selecting glucose oxidase as an enzyme to measure a glucose concentration in a sample solution has been put into practical use.

Meanwhile, in recent years, glycated proteins such as glycated hemoglobin and glycated albumin have been widely measured as an index for diabetes diagnosis. For example, hemoglobin A1c (hereinafter abbreviated as HbA1c) is one of glycated hemoglobins, and an HbAlc value is an inspection value which represents the ratio of hemoglobin bonded with sugar among hemoglobins in red blood cells. The HbAlc value reflects an average blood sugar level in the past one to two months. Therefore, the HbAlc value is less likely to be influenced by the pre-inspection meal as compared with a glucose level in blood, and is important as an index for diabetes control. The HbA1c value has been measured by an HPLC method or an immunization method using an optical spectroscopic method.

### PRIOR ART DOCUMENTS

### PATENT DOCUMENTS

Patent Document 1: JP-A-H03-202764

### SUMMARY OF THE INVENTION

### PROBLEMS TO BE SOLVED BY THE INVENTION

When the HbA1c value is measured, an inspection sample is diluted by, for example, about 100 times by a hemolysis treatment (hereinafter, in the present specification, a sample solution provided for measurement by a pretreatment such as a hemolysis treatment is referred to as a testing solution). Therefore, an HbAlc concentration in the testing solution is significantly reduced as compared with a glucose concentration, and is usually a low concentration on the order of µM. If an attempt is made to measure such a low HbAlc concentration by an electrochemical measurement method, the influence of a blank current becomes large, which makes it difficult to measure the low HbAlc concentration. Therefore, the method for measuring the HbA1c value using the electrochemical measurement method has not been put to practical use. If a background current can be reduced, the use of the electrochemical measurement method can be expected to allow the high-precision measurement of the HbAlc value. As a substance to be detected, proteins other than HbAlc such as hemoglobin, glycated albumin, glucose, cholesterol, lactic acid, and a ketone body (3-hydroxybutyric acid) allow high precision measurement if the blank current can be reduced.

Therefore, an object of the present invention is to provide a biological substance detection sensor used in an electrochemical measurement method and allowing high-precision measurement by reducing a blank current. It is another object of the present invention to provide a biological substance detection measurement kit and a component measuring method which allow high-precision measurement by reducing a blank current.

### SOLUTIONS TO THE PROBLEMS

In order to solve the above problems, the present inventors have conducted extensive studies. As a result, the present inventors have found that the inclusion of a halide or pseudo halide in a reagent part or a testing solution makes it possible to reduce a blank current, and have completed the present invention. That is, a biological substance detection sensor of the present invention is a biological substance detection sensor for analyzing a component in a testing solution using a mediator, the biological substance detection sensor comprising at least: an insulating substrate; a conductive part formed on the insulating substrate and including at least one pair of a working electrode and a counter electrode; and a reagent part disposed in contact with or in a vicinity of the conductive part and including at least one of protein and the mediator, the reagent part further including at least one additive selected from the group consisting of a halide and/or a pseudo halide.

A biological substance detection measurement kit of the present invention is a biological substance detection measurement kit used for an electrochemical-type biological substance detection sensor for analyzing a component in a testing solution using a mediator, the biological substance detection measurement kit comprising an additive to be added to the testing solution, the additive including at least one selected from the group consisting of a halide and/or a pseudo halide.

A method for measuring component of the present invention is a method using an electrochemical-type biological substance detection sensor for analyzing a component in a testing solution using a mediator, the method comprising a step of adding an additive to the testing solution, the additive including at least one selected from the group consisting of a halide and/or a pseudo halide.

### EFFECTS OF THE INVENTION

The present invention can provide a biological substance detection sensor, a biological substance detection measurement kit, and a method for measuring component which allow high-precision measurement by reducing a blank current.

### BRIEF DESCRIPTION OF THE DRAWINGS

Fig. 1 is an exploded perspective view showing an example of the structure of a biological substance detection sensor A according to an embodiment 1.
Fig. 2 is a vertical cross-sectional view taken along the longitudinal direction of the biological substance detection sensor A of Fig. 1.
Fig. 3 is a graph showing the results of Example 1, and shows the relationship between an additive and a blank current.
Fig. 4 is a graph showing the results of Example 2, and shows the relationship between an additive and a blank current.
Fig. 5 is a graph showing the results of Example 3, and shows the relationship between a glucose concentration and a detected current value.

### EMBODIMENTS OF THE INVENTION

Hereinafter, embodiments of the present invention will be described in detail with reference to the drawings and the like.

### Embodiment 1

The present embodiment relates to a biological substance detection sensor, which analyzes a component in a testing solution using a mediator. The biological substance detection sensor includes at least: an insulating substrate; a conductive part formed on the insulating substrate and including at least one pair of a working electrode and a counter electrode; and a reagent part disposed in contact with or in a vicinity of the conductive part and including at least one of protein and the mediator. The reagent part further includes at least one additive selected from the group consisting of a halide and/or a pseudo halide.

Examples of a biological substance to be detected by the biological substance detection sensor according to the present embodiment include hemoglobin, glycated hemoglobin, amino acid, glycated amino acid, glycated albumin, sugars such as glucose, vitamins such as vitamin C, alcohols, cholesterol, lactic acid, and a ketone body (3-hydroxybutyric acid).

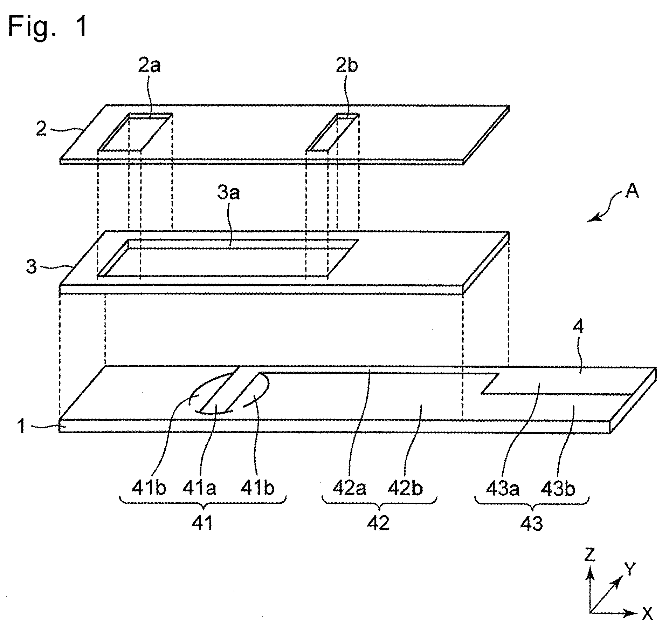

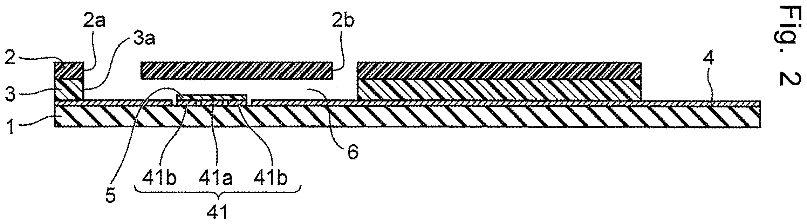

Fig. 1 is an exploded perspective view showing an example of the structure of a biological substance detection sensor A according to the present embodiment, and Fig. 2 is a vertical cross-sectional view taken along a longitudinal direction.

In the biological substance detection sensor A, an insulating substrate 1 including a conductive part 4 and a cover 2 are laminated with a spacer 3 interposed therebetween. Fig. 1 shows an example having a long rectangular piece shape having an X direction as a longitudinal direction and a Y direction as a width direction.

The conductive part 4 is formed on one main surface of the insulating substrate 1 having a pair of main surfaces facing each other. The conductive part 4 is a conductive portion, and includes a first electrode pair 41, a terminal part 43 formed on one end part of the insulating substrate 1, and a lead part 42 connecting the first electrode pair 41 and the terminal part 43 to each other. The first electrode pair 41 includes a working electrode 41a and a pair of counter electrodes 41b and 41b facing the working electrode 41a via a microvoid (described later) so as to sandwich the working electrode 41a in plan view. The working electrode 41a is connected to a terminal 43a via a lead 42a, and the counter electrodes 41b and 41b are connected to a terminal 43b via a lead 42b. Fig. 1 shows a structure excluding a reagent part described later. In Fig. 1, an example including only an electrode pair composed of a working electrode and a counter electrode is shown, but when are a plurality of substances to be detected are present in the testing solution, a plurality of electrode pairs can be provided on the insulating substrate according to the number of the substances to be detected.

The spacer 3 has an opening part 3a. The spacer 3 is shorter than the insulating substrate 1 so that the terminal part 43 is exposed. The cover 2 has two opening parts 2a and 2b along a longitudinal direction. The cover 2 is also shorter than the insulating substrate 1 so that the terminal part 43 is exposed, as in the case of the spacer 3. Here, the opening part of the spacer 3 and the opening parts of the cover 2 are disposed so that at least a part of the opening parts 2a and 2b of the cover 2 overlap with the opening part 3a of the spacer 3 when the cover 2 is stacked on the spacer 3.

Fig. 2 is a vertical cross-sectional view taken along the longitudinal direction of the biological substance detection sensor A shown in Fig. 1. The spacer 3 is sandwiched between the insulating substrate 1 and the cover 2, so that the opening part 3a of the spacer 3 forms a space part 6. A reagent part 5 is disposed on the first electrode pair 41. The reagent part 5 includes at least one of protein and a mediator, and at least one additive selected from the group consisting of a halide and/or a pseudo halide. The additive is included in the reagent part 5, which makes it possible to suppress the activity of the electrode causing an increase in a blank current value.

The opening part 2a of the cover 2 can be used as an opening for introducing the testing solution, and when the testing solution is added dropwise to the opening part 2a, the testing solution flows into the space part 6, where the testing solution is in contact with the reagent part 5. The opening part 2b of the cover 2 provides air into the space part 6, and functions to vent internal air with the drawing of the testing solution when the testing solution is added dropwise from the opening part 2a of the cover 2.

Examples of materials which can be used for the insulating substrate include, but are not particularly limited to, resin materials such as polyethylene terephthalate, polycarbonate, polyimide, polyethylene, polypropylene, polystyrene, polyvinyl chloride, polyoxymethylene, monomer cast nylon, polybutylene terephthalate, a methacrylic resin, and an ABS resin, and a glass material. Preferred are polyethylene terephthalate, polycarbonate, and polyimide, and more preferred is polyethylene terephthalate. The size of the insulating substrate is not particularly limited. For example, the insulating substrate has a total length of 5 to 100 mm, a width of 2 to 50 mm, and a thickness of 0.05 to 2 mm, preferably a total length of 7 to 50 mm, a width of 3 to 20 mm, and a thickness of 0.1 to 1 mm, and more preferably a total length of 10 to 30 mm, a width of 3 to 10 mm, and a thickness of 0.1 to 0.6 mm.

The conductive part on the insulating substrate can be formed by forming a conductive layer according to a sputtering method or a vapor deposition method or the like using, for example, carbon, gold, platinum, or palladium or the like as a material, and thereafter processing the conductive layer into a predetermined electrode pattern using a laser trimming method. Here, by forming a microvoid between the working electrode and the counter electrode, a microvoid between the leads, and a microvoid between the terminals according to the laser trimming method, electrical insulation between the electrodes, between the leads, and between the terminals is secured.

The material of the spacer is not particularly limited. For example, the same material as that of the insulating substrate can be used. The size of the spacer is also not particularly limited. For example, the spacer has a total length of 5 to 100 mm, a width of 2 to 50 mm, and a thickness of 0.01 to 1 mm, preferably a total length of 7 to 50 mm, a width of 3 to 20 mm, and a thickness of 0.05 to 0.5 mm, and more preferably a total length of 10 to 30 mm, a width of 3 to 10 mm, and a thickness of 0.05 to 0.25 mm.

The material of the cover is not particularly limited. For example, the same material as that of the insulating substrate can be used. The size of the cover is also not particularly limited. For example, the cover has a total length of 5 to 100 mm, a width of 3 to 50 mm, and a thickness of 0.01 to 0.5 mm, preferably a total length of 10 to 50 mm, a width of 3 to 20 mm, and a thickness of 0.05 to 0.25 mm, and more preferably a total length of 15 to 30 mm, a width of 5 to 10 mm, and a thickness of 0.05 to 0.1 mm. The cover preferably has a plurality of openings used as an air hole or a testing solution introducing port. As the shape of the opening, for example, a circle, an ellipse, or a polygon or the like can be used. For example, the opening has a maximum diameter of 0.01 to 10 mm, preferably a maximum diameter of 0.05 to 5 mm, and more preferably a maximum diameter of 0.1 to 2 mm. The opening may be formed by punching with a laser or a drill, or by using a mold.

The insulating substrate, the spacer, and the cover are laminated in this order, and j oined using an adhesive or heat fusion or the like for integrating, whereby the biological substance detection sensor can be produced. As the adhesive, an epoxy-based adhesive, an acrylic-based adhesive, a polyurethane-based adhesive, a hot-melt adhesive, and a UV curable adhesive and the like can be used.

The reagent part includes at least one of protein and a mediator, and at least one additive selected from the group consisting of a halide and/or a pseudo halide. The reagent part can include both the protein and the mediator, or only one of the protein and the mediator. When the reagent part includes only one of the protein and the mediator, the other can be used in a state where it is added into the testing solution before measurement.

Examples of the protein include an enzyme, an antibody, immune globulin, bovine serum albumin, and human serum albumin. Examples of the enzyme include oxidoreductases such as glucose oxidase, lactate oxidase, cholesterol oxidase, bilirubin oxidase, glucose dehydrogenase, lactate dehydrogenase, fructosyl amino acid oxidase, fructosyl peptide oxidase, and 3-hydroxybutyrate dehydrogenase. These oxidoreductases are oxidases or dehydrogenases which act on glucose, lactic acid, cholesterol, bilirubin, glycated amino acid, glycated peptide, and a ketone body (3-hydroxybutyric acid). The amount of the oxidoreductase is, for example, 0.01 to 100 U, preferably 0.05 to 10 U, and more preferably 0.1 to 5 U per sensor or per measurement.

Examples of the mediators include, but are not limited to, metal complexes (for example, an osmium complex, a ruthenium complex, and a ferric complex and the like), quinone compounds (for example, benzoquinone, naphthoquinone, phenanthrenequinone, phenanthrolinequinone, anthraquinone, and their derivatives and the like), a phenazine compound, a viologen compound, a phenothiazine compound, and a phenol compound. More specific examples thereof which are used include one or more compounds selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, a salt of 1,2-naphthoquinone-4-sulfonic acid, a salt of 9,10-phenanthrenequinone-2-sulfonic acid, a salt of 9,10-phenanthrenequinone-2,7-disulfonic acid, 1,10-phenanthroline-5,6-dione, a salt of anthraquinone-2-sulfonic acid, 1-methoxy-5-methylphenazinium methyl sulfate, methyl viologen, benzyl viologen, methylene blue, methylene green, 2-aminophenol, 2-amino-4-methylphenol, and 2,4-diaminophenol. Examples of the salts include, but are not limited to, a sodium salt, a potassium salt, a calcium salt, a magnesium salt, and a lithium salt. The amount of the mediator blended is not particularly limited, and the amount per measurement or per biological substance detection sensor is, for example, 0.1 pmol to 100 µmol, preferably 10 pmol to 10 µmol, and more preferably 50 pmol to 1 µmol.

As the additive, at least one selected from the group consisting of a halide and/or a pseudo halide is used. This additive is included in the reagent part, which makes it possible to reduce a blank current. As the halide, a chloride or bromide of an alkali metal or alkaline earth metal, or an ammonium salt which is a chloride or a bromide can be used. Specific examples thereof include NaCl, KC1, LiCl, MgCl₂, NaBr, KBr, LiBr, and MgBr₂. Preferred are NaBr, KBr, LiBr, and MgBr₂, and more preferred is NaBr or KBr. Examples of the ammonium salt include NH₄Cl and NH₄Br. As the pseudo halide, a thiocyanate, a cyanate, and an azide can be used. Specific examples thereof include NaSCN, NaOCN, tetrabutylammonium azide, 2-azido-1,3-dimethylimidazolinium hexafluorophosphate, and NaN₃. NaN₃ is preferred.

The amount of the additive blended is, for example, 1 pmol to 1000 µmol, preferably 50 pmol to 500 µmol, and more preferably 100 pmol to 100 µmol, per measurement or per biological substance detection sensor.

According to the present embodiment, at least one additive selected from the group consisting of a halide and/or a pseudo halide is included in the reagent part, whereby the blank current value can be reduced. As a result, an S/N ratio can be increased, whereby high-precision measurement is allowed in even the substance to be detected having a low concentration.

### Embodiment 2

The present embodiment relates to a biological substance detection measurement kit used for an electrochemical-type biological substance detection sensor for analyzing a component in a testing solution using a mediator. The biological substance detection measurement kit includes an additive to be added to the testing solution. The additive includes at least one selected from the group consisting of a halide and/or a pseudo halide.

The biological substance detection measurement kit according to the present embodiment (hereinafter, also referred to as a measurement kit) is used to treat the testing solution before measuring the component in the testing solution using the electrochemical-type biological substance detection sensor. The electrochemical-type biological substance detection sensor is not particularly limited as long as it can analyze the component in the testing solution using the mediator. Examples of the electrochemical-type biological substance detection sensor include one in which the reagent part includes no additive in the biological substance detection sensor of the embodiment 1. In this case, the protein and mediator described in the embodiment 1 can be used.

The measurement kit according to the present embodiment includes at least one additive selected from the group consisting of a halide and/or a pseudo halide. This additive is added to the testing solution, which makes it possible to reduce a blank current. As the halide, a chloride or bromide of an alkali metal or alkaline earth metal, or an ammonium salt which is a chloride or a bromide can be used. Specific examples thereof include NaCl, KCl, LiCl, MgCl₂, NaBr, KBr, LiBr, and MgBr₂. Preferred are NaBr, KBr, LiBr, and MgBr₂, and more preferred is NaBr or KBr. Examples of the ammonium salt include NH₄Cl and NH₄Br. As the pseudo halide, a thiocyanate, a cyanate, and an azide can be used. Specific examples thereof include NaSCN, NaOCN, tetrabutylammonium azide, 2-azido-1,3-dimethylimidazolinium hexafluorophosphate, and NaN₃. NaN₃ is preferred.

The additive is added to the testing solution so as to have a predetermined concentration. The concentration of the additive in the testing solution is 10 µM to 2 M, preferably 100 µM to 1 M, and more preferably 500 µM to 500 mM.

According to the present embodiment, at least one additive selected from the group consisting of a halide and/or a pseudo halide is added to the testing solution, whereby the blank current value can be reduced. As a result, an S/N ratio can be increased, whereby high-precision measurement is allowed in even the substance to be detected having a low concentration.

### Embodiment 3

The present embodiment relates to a method for measuring component using an electrochemical-type biological substance detection sensor for analyzing a component in a testing solution using a mediator. The method includes the step of adding an additive to the testing solution. The additive includes at least one selected from the group consisting of a halide and/or a pseudo halide.

The method for measuring component according to the present embodiment includes the step of treating the testing solution before measuring the component in the testing solution using the electrochemical-type biological substance detection sensor. The electrochemical-type biological substance detection sensor is not particularly limited as long as it can analyze the component in the testing solution using the mediator. Examples of the electrochemical-type biological substance detection sensor include one in which the reagent part includes no additive in the biological substance detection sensor of the embodiment 1. In this case, the protein and mediator described in the embodiment 1 can be used.

The method for measuring component according to the present embodiment includes the step of adding an additive to the testing solution prior to the measurement. The additive is at least one selected from the group consisting of a halide and/or a pseudo halide. This additive is added to the testing solution, which makes it possible to reduce a blank current. As the halide, a chloride or bromide of an alkali metal or alkaline earth metal, or an ammonium salt which is a chloride or a bromide can be used. Specific examples thereof include NaCl, KC1, LiCl, MgCl₂, NaBr, KBr, LiBr, and MgBr₂. Preferred are NaBr, KBr, LiBr, and MgBr₂, and more preferred is NaBr or KBr. Examples of the ammonium salt include NH₄Cl and NH₄Br. As the pseudo halide, a thiocyanate, a cyanate, and an azide can be used. Specific examples thereof include NaSCN, NaOCN, tetrabutylammonium azide, 2-azido-1,3-dimethylimidazolinium hexafluorophosphate, and NaN₃. NaN₃ is preferred.

The additive is added to the testing solution so as to have a predetermined concentration. The concentration of the additive in the testing solution is 10 µM to 2 M, preferably 100 µM to 1 M, and more preferably 500 µM to 500 mM.

According to the present embodiment, at least one additive selected from the group consisting of a halide and/or a pseudo halide is added to the testing solution, whereby the blank current value can be reduced. As a result, an S/N ratio can be increased, whereby high-precision measurement is allowed in even the substance to be detected having a low concentration.

### EXAMPLES

Hereinafter, the present invention will be described in more detail with reference to Examples, but the present invention is not limited to the following Examples.

### Example 1

In the present Example, a biological substance detection sensor of Fig. 1 including a conductive part formed of palladium and including no reagent part was used to investigate the influence of a halide and pseudo halide on a blank current. For comparison, sodium glutamate was used.

### (Experimental Method)

The following reagent solutions 1 and 2 were used.

### <Reagent Solution 1>

| | |
|---|---|
| Sodium phosphate buffer solution (pH: 6.5) | 25 mM |

### <Reagent Solution 2>

| | |
|---|---|
| Sodium phosphate buffer solution (pH: 6.5) | 25 mM |
| Additives | 0.5, 5, 50, 500mM |

The following reagents were used as additives.

Sodium chloride (NaCl), potassium chloride (KCl), lithium chloride (LiCl), magnesium chloride (MgCl₂), sodium bromide (NaBr), potassium bromide (KBr), potassium bromide hydrate (LiBr), bromide magnesium hexahydrate (MgBr₂), sodium thiocyanate (NaSCN), sodium azide (NaN₃) (all of which are manufactured by Wako Pure Chemical Industries, Ltd.), sodium L-glutamate (GluNa) (manufactured by Nacalai Tesque Inc.).

The reagent solution 1 or 2 was spotted on the biological substance detection sensor shown in Fig. 1 to perform electrochemical measurement. For the electrochemical measurement, the biological substance detection sensor was connected to a potentiostat manufactured by BAS Inc. A voltage of 0.4 V was applied between a working electrode and a counter electrode, and a current value after 15 seconds had elapsed was measured.

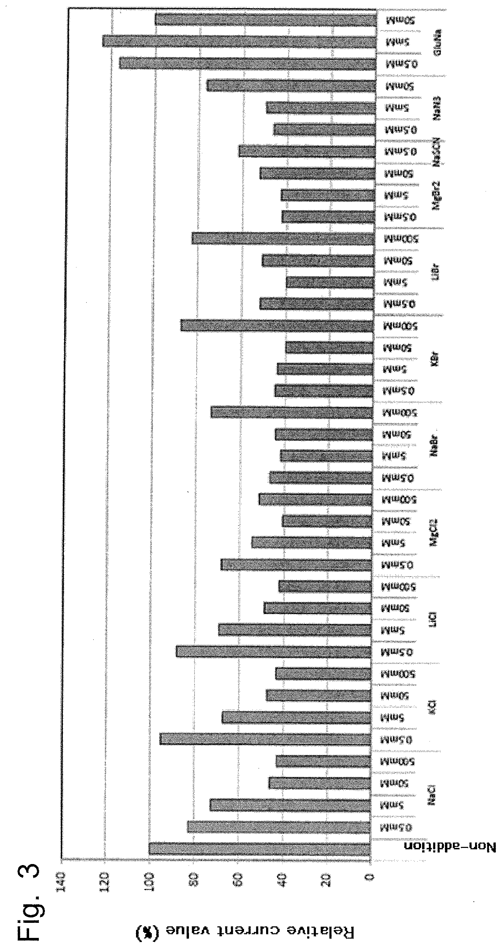

A current value when no additive was added was taken as 100%, and the ratio of a current value when each of the additives was added, to the current of 100% was calculated as a relative current value (%). The relative current value of each of the additives is shown in Fig. 3. Table 1 shows the relative current values when the concentrations of the additives are 0.5 mM.

**[Table 1]**

| Additives | Relative current values (%) when concentrations of additives are 0.5 mM |
|---|---|
| Non-addition | 100 |
| NaCl | 82.9 |
| KCl | 95.2 |
| LiCl | 88.2 |
| MgCl₂ | 68.2 |
| NaBr | 46.6 |
| KBr | 44.6 |
| LiBr | 51.6 |
| MgBr₂ | 42.0 |
| NaSCN | 62.0 |
| NaN₃ | 46.2 |
| GluNa | 116.2 |

The additives other than sodium glutamate significantly reduced the relative current value as compared with the case where no additive was added, and the effect of reducing the blank current was confirmed. It was found that a bromide, sodium thiocyanate, and sodium azide have a large effect of reducing the blank current even at a low concentration of 0.5 mM as compared with a chloride.

### Example 2

A blank current value was measured in the same manner as in Example 1 except that a biological substance detection sensor of Fig. 1 including a conductive part formed of platinum and including no reagent part was used.

### (Results)

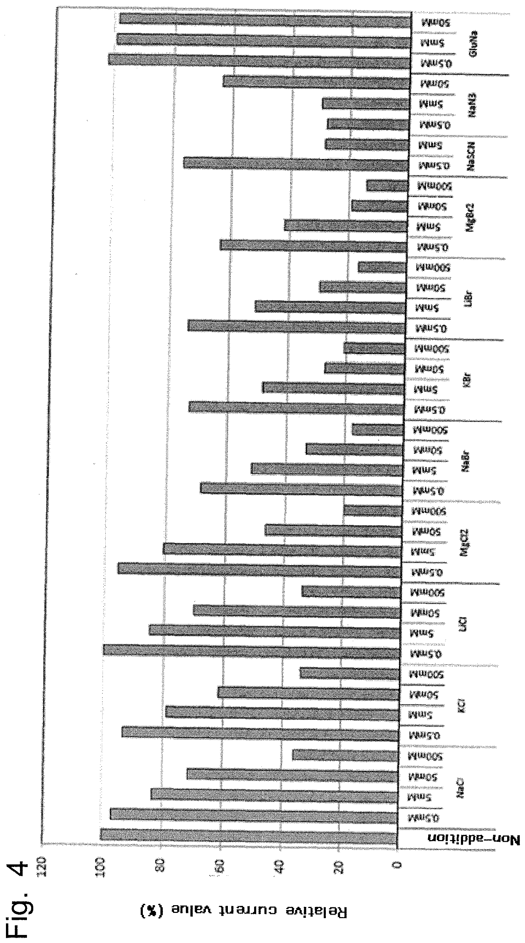

The relative current value of each of the additives is shown in Fig. 4. Table 2 shows the relative current values when the concentrations of the additives are 5 mM.

**[Table 2]**

| Additives | Relative current values (%) when concentrations of additives are 5 mM |
|---|---|
| Non-addition | 100 |
| NaCl | 83.4 |
| KCl | 78.8 |
| LiCl | 84.7 |
| MgCl₂ | 80.5 |
| NaBr | 51.5 |
| KBr | 48.2 |
| LiBr | 51.1 |
| MgBr₂ | 41.8 |
| NaSCN | 28.4 |
| NaN₃ | 29.8 |
| GluNa | 99.4 |

The additives other than sodium glutamate significantly reduced the relative current value as compared with the case where no additive was added, and the effect of reducing the blank current was confirmed. As in the case of Example 1, it was found that a bromide, sodium thiocyanate, and sodium azide have a large effect of reducing a blank current even at a low concentration of 5 mM as compared with a chloride. In particular, sodium azide exhibited a remarkable effect at low concentrations.

### Example 3

### <Production of Biological Substance Detection Sensor>

The surface of a first electrode pair on an insulating substrate constituting a biological substance detection sensor A of Fig. 1 and including a conductive part formed of palladium was coated with 4 µL of the following reagent solution 3, followed by drying at a temperature of 25°C and a humidity of 50% for about 3 hours, thereby forming a reagent part. A biological substance detection sensor shown in Fig. 1 was produced.

### <Reagent Solution 3>

| | |
|---|---|
| Sodium phosphate buffer solution (pH 8.0) | 5mM |
| Glucose dehydrogenase (GDHGLD1) | 2000U/mL |
| n-dodecyl-β-D-maltoside | 0.003%(wt/v) |

Here, as glucose dehydrogenase, Glucose Dehydrogenase (FAD-dependent) manufactured by BBI International Inc. was used. n-dodecyl-β-D-maltoside manufactured by Dojindo Laboratories was used.

A testing solution having the following composition was spotted on the biological substance detection sensor to perform electrochemical measurement. In the electrochemical measurement, the biological substance detection sensor A was connected to a potentiostat. A voltage of 0.4 V was applied between a working electrode and a counter electrode, and a current value after 10 seconds had elapsed was measured.

### <Testing Solution>

| | |
|---|---|
| Sodium phosphate buffer solution (pH 8.0) | 25mM |
| Sodium 9,10-phenanthrenequinone-2-sulfonate (PQSA) | 8mM |
| Glucose | 0, 100, 1000µM |
| Additives | 1mMor 100mM |

Sodium chloride, potassium bromide, and sodium azide were used as the additives. Sodium 9,10-phenanthrenequinone-2-sulfonate was obtained by sulfonation of commercially available 9,10-phenanthrenequinone with fuming sulfuric acid, separation of isomers, and Na salification.

### (Results)

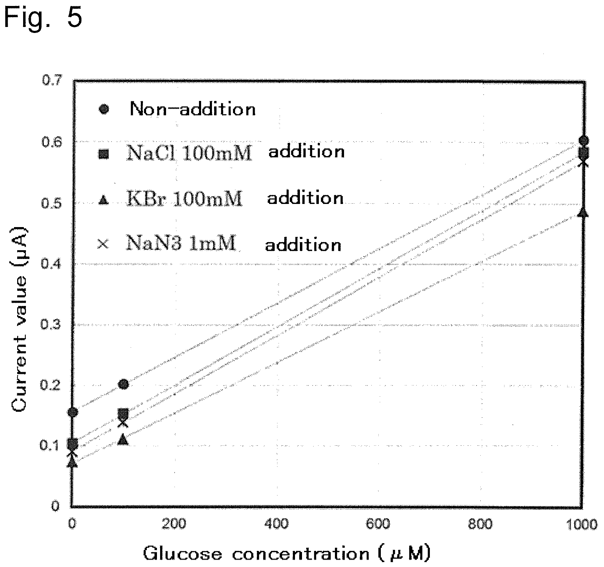

Fig. 5 shows the relationship between a glucose concentration and a detected current value. It is found that the presence of the additive reduces the blank current value as compared with the case where no additive is added. A linear relationship was obtained in the range of 0 to 1000 µM. S/N ratios (detected current value/blank current value) at glucose concentrations of 100 µM and 1000 µM are calculated, and shown in Table 3. It could be confirmed that the S/N ratio can be improved as compared with the case where no additive is added.

**[Table 3]**

| | S/N ratio (glucose: 100 µM) | S/N ratio (glucose: 1000 µM) |
|---|---|---|
| Non-addition | 1.30 | 3.88 |
| NaCl 100mM | 1.47 | 5.63 |
| KBr 100mM | 1.49 | 6.63 |
| NaN3 1mM | 1.54 | 6.34 |

### INDUSTRIAL APPLICABILITY

The present invention can provide a biological substance detection sensor, a biological substance detection measurement kit, and a method for measuring component which allow high-precision measurement.

### DESCRIPTION OF REFERENCE SIGNS

- 1:: Insulating substrate
- 2:: Cover
- 2a, 2b:: Cover opening part
- 3:: Spacer
- 3a:: Spacer opening part
- 4:: Conductive part
- 41:: First electrode pair
- 41a:: Working electrode
- 41b:: Counter electrode
- 42:: Lead part
- 42a, 42b:: Lead
- 43:: Terminal part
- 43a, 43b:: Terminal
- 5:: Reagent part
- 6:: Space part

## Claims

1. A biological substance detection sensor for analyzing a component in a testing solution using a mediator, comprising at least:
an insulating substrate;
a conductive part formed on a surface of the insulating substrate and including at least one pair of a working electrode and a counter electrode; and
a reagent part disposed in contact with or in a vicinity of the conductive part and including at least one of protein and the mediator, the reagent part further including at least one additive selected from the group consisting of a halide and/or a pseudo halide.

2. The biological substance detection sensor according to claim 1, wherein the halide is a chloride or bromide of an alkali metal or alkaline earth metal, or an ammonium salt which is a chloride or a bromide, and wherein the pseudo halide is a thiocyanate, a cyanate, and an azide.

3. The biological substance detection sensor according to claim 1 or 2, wherein the reagent part includes the protein and the additive.

4. The biological substance detection sensor according to claim 3, wherein the protein is an oxidoreductase.

5. The biological substance detection sensor according to claim 4, wherein the oxidoreductase is an oxidase or a dehydrogenase which acts on glucose, glycated amino acid, or glycated peptide.

6. A biological substance detection measurement kit used for an electrochemical-type biological substance detection sensor for analyzing a component in a testing solution using a mediator, comprising an additive to be added to the testing solution, the additive including at least one selected from the group consisting of a halide and/or a pseudo halide.

7. The biological substance detection measurement kit according to claim 6, wherein the halide is a chloride or bromide of an alkali metal or alkaline earth metal, or an ammonium salt which is a chloride or a bromide, and wherein the pseudo halide is a thiocyanate, a cyanate, and an azide.

8. The biological substance detection measurement kit according to claim 6 or 7, wherein the electrochemical-type biological substance detection sensor comprises at least:
an insulating substrate;
a conductive part formed on a surface of the insulating substrate and including at least one pair of a working electrode and a counter electrode; and
a reagent part disposed in contact with or in a vicinity of the conductive part and including at least one of protein and the mediator.

9. The biological substance detection measurement kit according to claim 8, wherein the protein is an oxidoreductase.

10. The biological substance detection measurement kit according to claim 9, wherein the oxidoreductase is an oxidase or a dehydrogenase which acts on glucose, glycated amino acid, or glycated peptide.

11. A method for measuring component using an electrochemical-type biological substance detection sensor for analyzing a component in a testing solution using a mediator, comprising a step of adding an additive to the testing solution, the additive including at least one selected from the group consisting of a halide and/or a pseudo halide.

12. The component measuring method according to claim 11, wherein the halide is a chloride or bromide of an alkali metal or alkaline earth metal, or an ammonium salt which is a chloride or a bromide, and wherein the pseudo halide is a thiocyanate, a cyanate, and an azide.

13. The component measuring method according to claim 11 or 12, wherein the electrochemical-type biological substance detection sensor comprises at least:
an insulating substrate;
a conductive part formed on a surface of the insulating substrate and including at least one pair of a working electrode and a counter electrode; and
a reagent part disposed in contact with or in a vicinity of the conductive part and including at least one of protein and the mediator.

14. The component measuring method according to claim 13, wherein the protein is an oxidoreductase.

15. The component measuring method according to claim 14, wherein the oxidoreductase is an oxidase or a dehydrogenase which acts on glucose, glycated amino acid, or glycated peptide.
